Europäisches Patentamt

European Patent Office

Office européen des brevets

(19)

(11) **EP 1 598 766 A2**

(12) # EUROPEAN PATENT APPLICATION

(43) Date of publication:
**23.11.2005 Bulletin 2005/47**

(51) Int Cl.7: **G06F 19/00**

(21) Application number: **05010236.7**

(22) Date of filing: **11.05.2005**

(84) Designated Contracting States:
**AT BE BG CH CY CZ DE DK EE ES FI FR GB GR
HU IE IS IT LI LT LU MC NL PL PT RO SE SI SK TR**
Designated Extension States:
**AL BA HR LV MK YU**

(30) Priority: **11.05.2004 JP 2004141721**

(71) Applicant: **SYSMEX CORPORATION
Kobe-shi, Hyogo 651-0073 (JP)**

(72) Inventors:
• **Kouchi, Yasuhiro
Kakogawa-shi Hyogo 675-0061 (JP)**

• **Saitou, Takeo
Kobe-shi Hyogo 651-2111 (JP)**
• **Naitou, Yasuhiro
Fujisawa-shi Kanagawa 252-0816 (JP)**
• **Nakajima, Hiromu
Osaka-shi Osaka 545-0014 (JP)**

(74) Representative: **HOFFMANN EITLE
Patent- und Rechtsanwälte
Arabellastrasse 4
81925 München (DE)**

(54) **Simulation system for simulating material concentration in a living body and storage medium**

(57)     A system for computer simulation of object material concentration in a living body using a biological simulation model configured by a blocks readily corresponding to organs of a living body is provided. The functions of organs in a living body related to the object material are described using the biological simulation model to simulate the change over time in the object material concentration in a living body using a computer. The blocks are mutually linked so as to enable the transference of data among blocks. A computer-readable storage medium is also disclosed.

EP 1 598 766 A2

**Description**

FIELD OF THE INVENTION

The present invention relates to a system for simulating material concentrations in living tissue using a computer, and relates to a storage medium for recording a computer program for having a computer function as such a system.

BACKGROUND

Numerical models have been tried heretofore to determine material concentration in living tissue, particularly blood glucose and blood insulin levels from the perspective of medical use in the diagnosis of diabetes.

The models used include, for example, Bergman's minimal model (Bergman et al., American Journal of Physiology, vol. 236(6), p. E-667-77 (1979), and Bergman et al., Journal of Clinical Investigation, vol. 68(6), p. 1456-67 (1981)). This minimal model numerically represents plasma glucose concentration, plasma insulin concentration, and amount of acting insulin at the insulin action site of peripheral tissue, that is, remote insulin, as variables. In this case, when the plasma glucose concentration at time t is designated G(t), plasma insulin concentration is designated I(t), and remote insulin is designated X(t), then G(t), I(t), and X(t) the respective time differentials can be described on the left side of the differential equations below.

$$dG(t) / dt = -p_1 (G(t)- G_b )-X(t)G(t)$$

$$dX(t) / dt = -p_2 X(t) + p_3(I(t)-I_b)$$

$$dI(t) / dt = -n(I(t) - I_b) + \gamma(G(t) - h) \text{ where } G(t) > h$$

$$= -n(I(t) - I_b) + \gamma(G(t) - h) \text{ where } G(t) <= h$$

The parameters in the equations are listed below and can be set at different values depending on the individual.

$p_1$ : Non-insulin-dependent glucose metabolism rate
$G_b$ : Basal glucose concentration
$p_2$ : Insulin uptake at insulin action site
$p_3$ : Insulin consumption rate relative to insulin-dependent glucose metabolism
$I_b$ : Basal insulin concentration
n : Insulin consumption per unit time
$\gamma$ : Insulin secretion sensitivity relative to glucose simulation
h : Blood glucose threshold value at which insulin secretion begins

Generally, in living bodies, blood glucose is regulated by four mutually inter-related blocks including the pancreas which secretes insulin in accordance with blood glucose stimulation, the liver which produces glucose into the blood or takes up glucose from the blood in accordance with insulin and blood glucose levels, circulation system kinetics which distribute insulin to peripheral tissues, and peripheral tissues which are acted upon by the insulin and metabolize the insulin. In the minimal model, the structural elements of the model are abstract elements which do not correspond to the four blocks of living bodies, which presents difficulties when applying the simulated results of living body blood glucose fluctuation and insulin level fluctuation to the four inter-related blocks of the living body.

Other blood glucose level reproduction methods include methods of predicting blood glucose level of a diabetic patient (for example, refer to Japanese Laid-Open Patent Publication No. 11-296598). Although it is possible to predict blood glucose levels according to this method, it is not possible to know the condition of organs participating in the regulation of blood glucose.

SUMMARY

The scope of the present invention is defined solely by the appended claims, and is not affected to any degree by the statements within this summary.

In view of the information described above, an object of the present invention is to provide a system for computer simulation of function in a living body using a biological simulation model configured by a program readily corresponding to structural elements of a living body.

The first aspect of the present invention relates to a simulation system comprising a biological simulation model representing the functions of organs in a living body related to a object material, and having a plurality of blocks corresponding to the organs respectively, which are mutually linked so as to enable the transference of data among blocks, and a calculation means for successively calculating the object material concentration in the living body to simulate the activity of the organs, and simulating the activity of the organs by driving the biological simulation model.

The second aspect of the present invention relates to a computer-readable storage medium for recording a computer program for working a computer as a system for simulating a change over time in object material concentration in a living body, the computer program comprising a step of driving, in a computer, a biological simulation model representing the functions of organs in a living body related to the object material to successively calculate the object material concentration in the living body, and wherein the biological simulation model have a plurality of blocks corresponding to the organs respectively, which are mutually linked so as to enable the transference of data among blocks.

BRIEF DESCRIPTION OF THE DRAWINGS

Fig. 1 is a block diagram showing the hardware structure of the simulation system of an embodiment of the present invention;

Fig. 2 is a function block diagram showing the general structure of the models of the embodiment of the present invention;

Fig. 3 is a block drawing showing the structure of the pancreas model shown in Fig. 2;

Fig. 4 is a block drawing showing the structure of the liver model shown in Fig. 2;

Fig. 5 is a block drawing showing the structure of the insulin kinetics model shown in Fig. 2;

Fig. 6 is a block drawing showing the structure of the peripheral tissue model shown in Fig. 2;

Fig. 7 is a graph showing an example of glucose absorption rate used as the input in the embodiment of the present invention;

Fig. 8 is a graph showing and example of blood glucose level reproduced in the embodiment of the present invention;

Fig. 9 is a graph showing an example of the peripheral tissue insulin level reproduced in the embodiment of the present invention;

Fig. 10 is a graph showing an example of blood insulin level reproduced in the embodiment of the present invention; and

Fig. 11 is a graph showing an example of clinically measured blood glucose levels.

DETAILED DESCRIPTION OF THE EMBODIMENT

An embodiment of the present invention is described hereinafter based on the drawings.

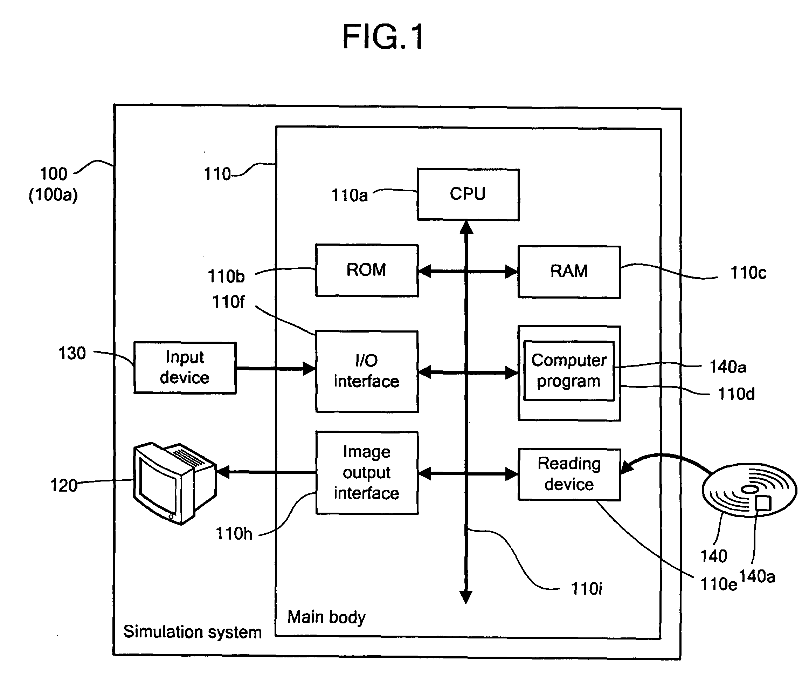

Fig. 1 is a block diagram showing the hardware structure of the simulation system of an embodiment of the present invention. A simulation system 100 of an embodiment of the present invention is configured by a computer 100a, which mainly includes a body 110, display 120, and input device 130. The body 110 is mainly configured by a CPU 110a, ROM 110b, RAM 110c, hard disk 110d, reading device 110e, I/O interface 110f, communication interface 110g, and image output interface 110h. The CPU 110a, ROM 110b, RAM 110c, hard disk 110d, reading device 110e, I/O interface 110f, and image output interface 110h are connected by a bus 110i capable of data communication.

The CPU 110a is capable of executing computer programs loaded in RAM 110c and computer programs stored in ROM 110b. Each of the function blocks, which are described later, are realized by the CPU 110a executing an application program 140a, also described later, such that the computer 100a functions as the simulation system 100.

The ROM 110b may be mask ROM, PROM, EPROM, EEPROM or the like, and stores computer programs executed by the CPU 110a and data used by the computer programs.

The RAM 110c may be SRAM, or DRAM or the like. The RAM 110c is used to read the computer programs recorded on the hard disk 110d and ROM 110. Furthermore, the RAM 110c is used as an operation area of the CPU 110a when these computer programs are executed.

The hard disk 110d is used for installing an operating system and applications programs and the like, various types of computer programs executed by the CPU 110a and data used in the execution of the computer programs. The application program 140a, which is described later, is also installed on the hard disk 110d.

The reading device 110e may be a flexible disk drive, CD-ROM drive, DVD-ROM drive or the like, capable of reading data and computer programs recorded on a portable storage medium 140. Furthermore, the application program 140a which enables a computer to function as the simulation system of the present invention is stored on the portable storage medium 140, and the application program 140a of the present invention can be read from the portable storage medium 140 by the computer 100a and the application program 140a can be installed on the hard disk 110d.

The application program 140a need not be provided on the portable storage medium 140, inasmuch as the application program 140a also may be provided over an electric communication line from an external device connected to the computer 100a so as to enable communication by means of an electric communication line (wire line or wireless). For example, the application program 140a may be stored on the hard disk or a server computer connected to the internet, such that the computer 100a can access the server computer and download computer programs and the like which are then installed on the hard disk 110d.

An operating system providing a graphical user interface environment, such as, for example, Windows (registered trademark), a commercial product of Microsoft Corporation of the USA, or the like is installed on the hard disk 110d. In the following description, the application program 140a of the present embodiment operates in such an operating system.

The I/O interface 110f may be, for example, a serial interface such as a USB, IEEE1394, RS-232C or the like, a parallel interface such as SCSI, IDE, IEEE1284 or the like, or an analog interface such as a D/A converter, A/D converter or the like. The I/O interface 110f is connected to an input device 130 configured by a keyboard and mouse, such that a user may input data to the computer 100a using the input device 130.

The image output interface 110h is connected to a device 120 such as an LCD, CRT or the like, so as to output image signals corresponding to image data received from the CPU 110a on the display 120. The display 120 displays the images (screens) in accordance with the input image signals.

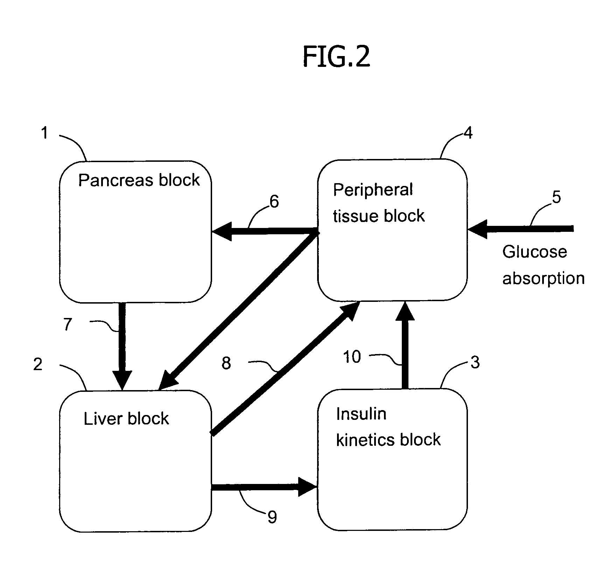

Fig. 2 is a function block diagram showing the general structure of the models of the embodiment of the present invention. As shown in Fig. 2, a living body model used in the simulation of the present invention is configured by a pancreas block 1, liver block 2, insulin kinetics block 3, and peripheral tissue block 4, in which each block has respective inputs and outputs.

The pancreas block 1 has blood glucose level 6 as an input, and insulin secretion rate 7 as an output. The liver block 2 has blood glucose level 6 and insulin secretion rate 7 as inputs, and net glucose production 8 and liver-passed insulin 9 as outputs. The insulin kinetics block 3 has liver-passed insulin 9 as an input, and insulin level 10 in the peripheral tissue as an output. The peripheral tissue block 4 has net glucose production 8, external glucose absorption 5, and insulin level 10 in the peripheral tissue as inputs, and blood glucose level 6 as an output. Glucose absorption 5 is externally obtained data, and this function can be realized, for example, by a user inputting testing data and the like using the input device 130. The function blocks 1-4 may be realized by the CPU 100a executing the computer program 140a.

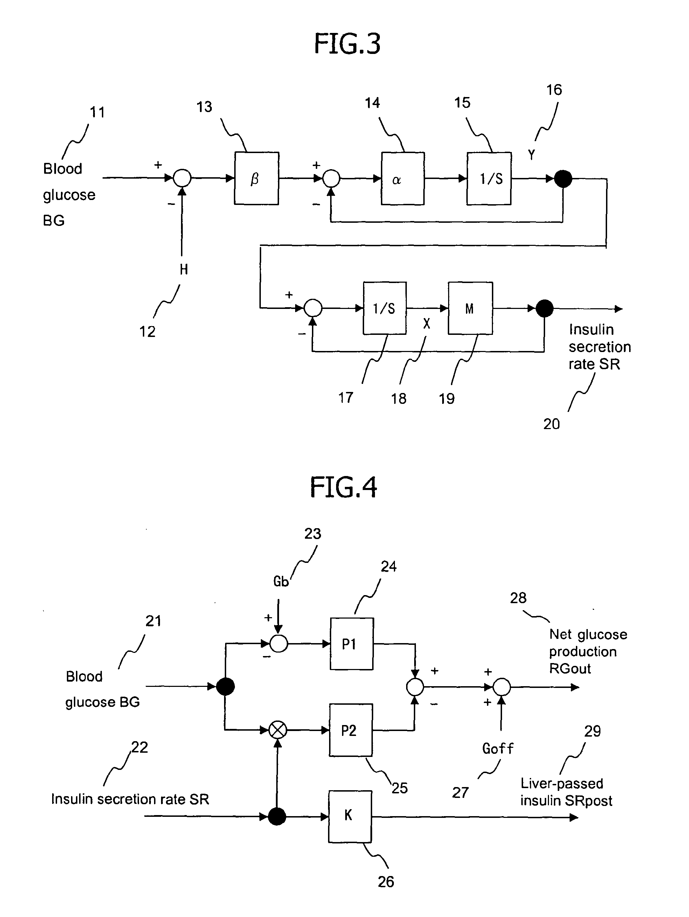

Details of each block in the present embodiment are shown below. The relationship between inputs and outputs in pancreas block 1 are described using the differential equation 1 shown below. Furthermore, the relationship can be realized using the block drawing shown in Fig. 3, which is equivalent to differential equation 1.

$$\text{Differential Equation 1}$$

$$dY/dt = -\alpha\{Y(t)-\beta(BG(t)-h)\} \quad (\text{where } BG(t)>h)$$

$$= -\alpha Y(t) \quad (\text{where } BG(t)<=h)$$

$$dX/dt = -M\dot{}X(t)+Y(t)$$

$$SR(t) = M\dot{}X(t)$$

Where the variables and the parameters in the differential equation 1 are defined as follows, and each parameter can represent different values for each patient.

Variables

BG(t): Blood glucose level
X(t): Total insulin secretable from the pancreas
Y(t): Delivery rate of newly delivered insulin under glucose stimulation
SR(t): Insulin secretion rate from the pancreas

Parameters

h: Glucose rate threshold capable of stimulating insulin delivery
$\alpha$: Follow-up under glucose stimulation
$\beta$: Sensitivity to glucose stimulation
M: Secretion rate per unit concentration

In this case, the blood glucose level 6, which is input to the pancreas block 1 in Fig. 2, corresponds to BG(t). The insulin secretion rate 7, which is output from the pancreas block 1 in Fig. 2, corresponds to SR(t).

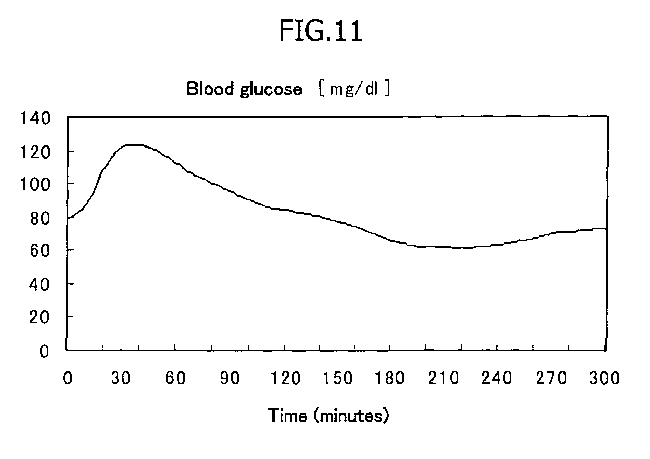

In the block drawing of Fig. 3, 11 refers to the blood glucose level (blood glucose) BG, 12 refers to the glucose level threshold H capable of stimulating insulin delivery, 13 refers to sensitivity $\beta$ to glucose stimulation, 14 refers to follow-up $\alpha$ to glucose stimulation, 15 refers to integral elements, 16 refers to insulin delivery rate Y of insulin newly delivered under glucose stimulation, 17 refers to integral elements, 18 refers to total insulin X secretable from the pancreas, 19 refers to secretion rate M per unit concentration, and 20 refers to insulin secretion rate SR from the pancreas.

The relationship between inputs and outputs in liver block 2 are described using the differential equation 2 shown below. Furthermore, the relationship can be realized using the block drawing shown in Fig. 4, which is equivalent to differential equation 2.

Differential Equation 2

$$RGout(t) = P_1(G_b - BG(t)) - P_2 \cdot SR(t) \cdot BG(t) + G_{off} \text{ (where BG}(t) < G_b)$$

$$= -P_2 \cdot SR(t) \cdot BG(t) + G_{off} \text{ (otherwise)}$$

$$SRpost(t) = K \cdot SR(t)$$

Where the variables and the parameters in the differential equation 2 are defined as follows, and each parameter can represent different values for each patient.

Variables

BG(t): Blood glucose level
SR(t) Insulin secretion rate from the pancreas
RGout(t): Net glucose from the liver
SRout(t): Liver-passed insulin

Parameters

$G_b$ : Basal glucose concentration

$P_1$ : Glucose production rate below glucose stimulation $G_b$
$P_2$ : Glucose uptake rate per unit insulin, unit glucose by the liver
K : Insulin uptake percentage by the liver
$G_{off}$ : Glucose production rate relative to base metabolism

In this case, the blood glucose level 6, which is input to the liver block in Fig. 2, corresponds to BG(t), and the insulin secretion rate 7 corresponds to SR(t). The net glucose production 8, which is output from the liver block in Fig. 2, corresponds to RGout(t), and the liver-passed insulin 9 corresponds to SRout(t).

In the block drawing of Fig. 4, 21 refers to blood glucose level (blood glucose value) BG, 22 refers to insulin secretion rate SR from the pancreas, 23 refers to the basal glucose concentration $G_b$, 24 refers to the glucose production rate $P_1$ below glucose stimulation $G_b$, 25 refers to glucose uptake rate $P_2$ per unit glucose and unit insulin by the liver, 26 refers to the insulin uptake rate K by the liver, 27 refers to the glucose production rate $G_{off}$ relative to the base metabolism, 28 refers to the net glucose RGout from the liver, and 29 refers to liver-passed insulin SRpost.

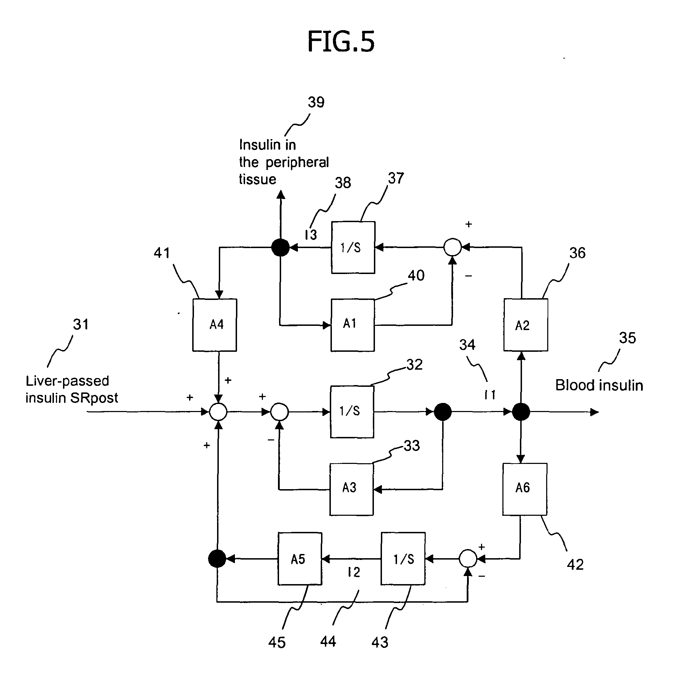

The relationship between inputs and outputs in insulin kinetic block 3 are described using the differential equation 3 shown below. Furthermore, the relationship can be realized using the block drawing shown in Fig. 5, which is equivalent to differential equation 3.

Differential Equation 3

$$dI_1(t)/dt = -A_3 I_1(t) + A_5 I_2(t) + A_4 I_3(t) + SRpost(t)$$

$$dI_2(t)/dt = A_6 I_1(t) - A_5 I_2(t)$$

$$dI_3(t)/dt = A_2 I_1(t) - A_1 I_3(t)$$

Where the variables and the parameters in the differential equation 3 are defined as follows, and each parameter can represent different values for each patient.

Variables

SRpost(t) : Liver-passed insulin

$I_1(t)$ :Blood insulin level

$I_2(t)$ :Insulin level in the insulin-independent tissue

$I_3(t)$ :Insulin level in the peripheral tissue

Parameters

$A_1$ :Insulin loss rate in the peripheral tissues
$A_2$ :Insulin distribution rate to the peripheral tissue
$A_3$ :Liver-passed insulin distribution rate
$A_4$ :Peripheral tissue-passed insulin outflow rate
$A_5$ :Insulin loss rate in the insulin-independent tissue
$A_6$ :Insulin distribution rate to the insulin-independent tissue

In this case, the liver-passed insulin 9, which is input to the insulin kinetic block in Fig. 2, corresponds to SRout (t). The peripheral tissue insulin level 10, which is output from the insulin kinetic block in Fig. 2, corresponds to $I_3$ (t).

In the block drawing of Fig. 5, 31 refers to liver-passed insulin SRpost, 32 refers to integral element, 33 refers to liver-passed insulin distribution rate A3, 34 and 35 refer to blood insulin level I1, 36 refers to insulin distribution rate A2 to the peripheral tissue, 40 refers to insulin loss rate $A_1$ in the peripheral tissue, 41 refers to peripheral tissue-passed insulin outflow rate $A_4$, 42 refers to insulin distribution rate $A_6$ to the insulin-independent tissue, 43 refers to an integral element, 44 refers to insulin level $I_2$ in the insulin-independent tissue, and 45 refers to insulin loss rate $A_5$ in the insulin-independent tissue.

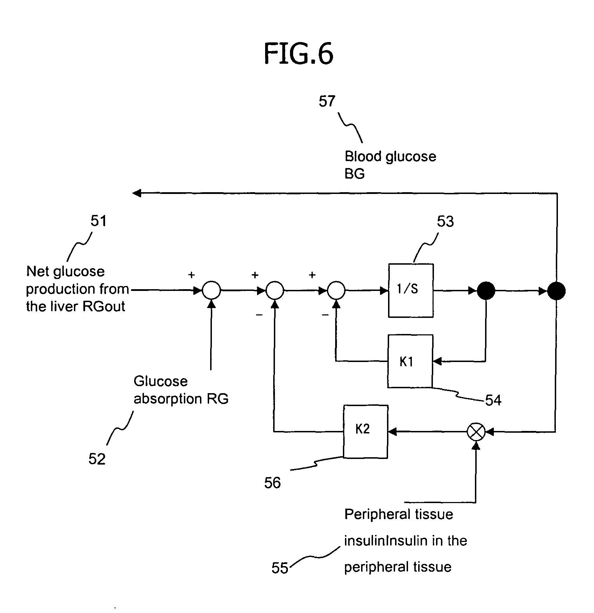

The relationship between inputs and outputs in peripheral tissue block 4 are described using the differential equation 4 shown below. Furthermore, the relationship can be realized using the block drawing shown in Fig. 6, which is equivalent to differential equation 4.

Differential Equation 4

$$dBG(t)/dt = -K_1 \cdot BG(t) - K_2 \cdot I_3(t) \cdot BG(t) + RG(t) + RGout(t)$$

Where the variables and the parameters in the differential equation 4 are defined as follows, and each parameter can represent different values for each patient.

Variables

BG(t) : Blood glucose level

RG(t) :Glucose absorption from alimentary canal

RGout(t) :Net glucose from the liver

$I_3(t)$ :Insulin level in the peripheral tissue

Parameters

$K_1$ :Non-insulin-dependent glucose consumption rate in the peripheral tissue
$K_2$ :Insulin-dependent glucose consumption rate in the peripheral tissue

In this case, the insulin level 10 in the peripheral tissue, which is input to the peripheral tissue block in Fig. 2, corresponds to $I_3(t)$, the net glucose 8 from the liver corresponds to RGout(t), and glucose absorption 5 from the alimentary canal corresponds to RG(t). The blood glucose level 6, which is output from the peripheral tissue block in Fig. 2, corresponds to BG(t).

In the block drawing of Fig. 6, 51 refers to the net glucose RGout from the liver, 52 refers to the glucose absorption RG from the alimentary canal, 53 refers to integral element, 54 refers to the insulin-independent glucose consumption rate $K_1$ in the peripheral tissue, 55 refers to the insulin level $I_3$ in the peripheral tissue, 56 refers to the insulin-dependent glucose consumption rate $K_2$ in the peripheral tissue, and 57 refers to the blood glucose level BG.

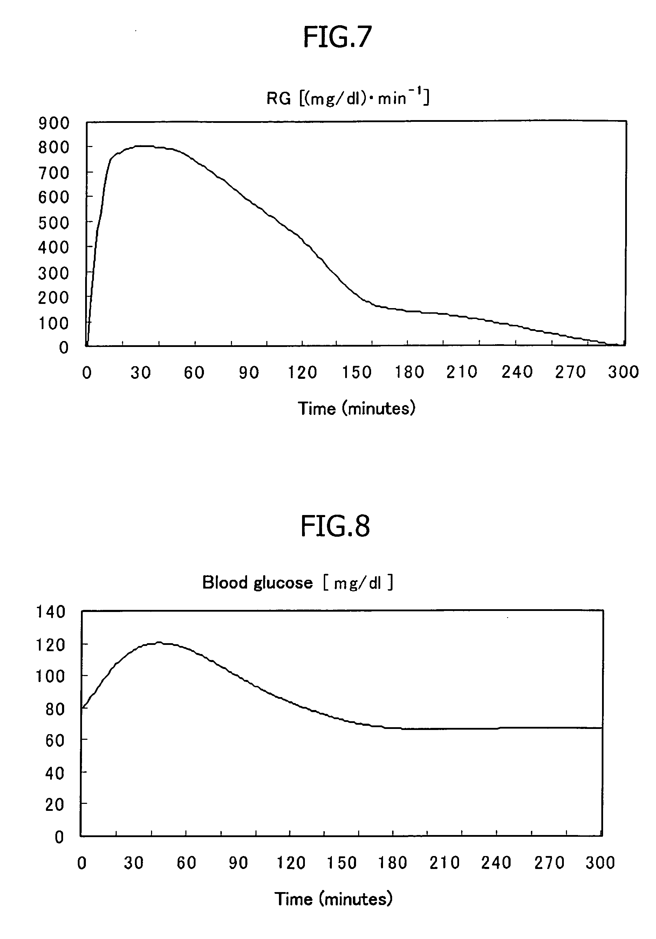

Then, the operation of the simulation system of the embodiment of the present invention is explained. FIG.7 is a flow chart showing the flow of the operation of the simulation system of the embodiment of the present invention.

At first, a user performs predetermined operation to the input device 130, such as double-clicking to the corresponding icon, instructs the computer 100a to execute the application program 140a. By this, Application program 140a is read-outed from hard disk 110d, and is loaded into RAM110c. After starting the application program 140a, the time-series data of glucose absorption 5 (i.e. intake of glucose) from digestive organs, glucose concentration (i.e. blood glucose level), and insulin concentration, which provided by OGTT (Oral Glucose-Tolerance Test), are inputted into computer 100a by the user.

The CPU 110a accepts the input of these data (step S1), and implements the parameter values generation process (step S2). In the parameter values generation process S2, the appropriate parameter values for the model are computed. When those appropriate parameter values are set to the model, the time-series data of glucose concentration and insulin concentration approximated to the inputted data is obtained as a simulation result. The genetic algorithm is used for the parameter values generation process S2 of this embodiment, and this process S2 is able to compute the group of the parameter values which obtain the simulation result data most approximated to input data. In addition, Besides the genetic algorithm, a well-known other algorithm such as the least-squares method, the steepest descent method or the simulated annealing method may be used as a calculation method of parameter values.

Subsequently, the CPU110a sets a group of parameter values produced by parameter values generation process S2 to the living body simulation model (step S3), and implements the simulation process (step S4). In the simulation process S4, the glucose absorption 5 is inputted into the living body simulation model, and the insulin concentration 10 and blood glucose concentration 6 which correspond to the glucose absorption 5 is calculated. In this process, the glucose absorption 5 in step S1 may be used, and the time-series data of glucose absorption inputted separately from step S1 may be used. In addition, time-series data of glucose absorption produced by CPU110a automatically may be used. As shown in Fig. 2, since the inputs and outputs among the various blocks configuring the system are mutually connected for data transfers, the change over time in blood glucose and insulin levels can be calculated based on the equations to effect simulation by the glucose absorption 5 from the alimentary canal.

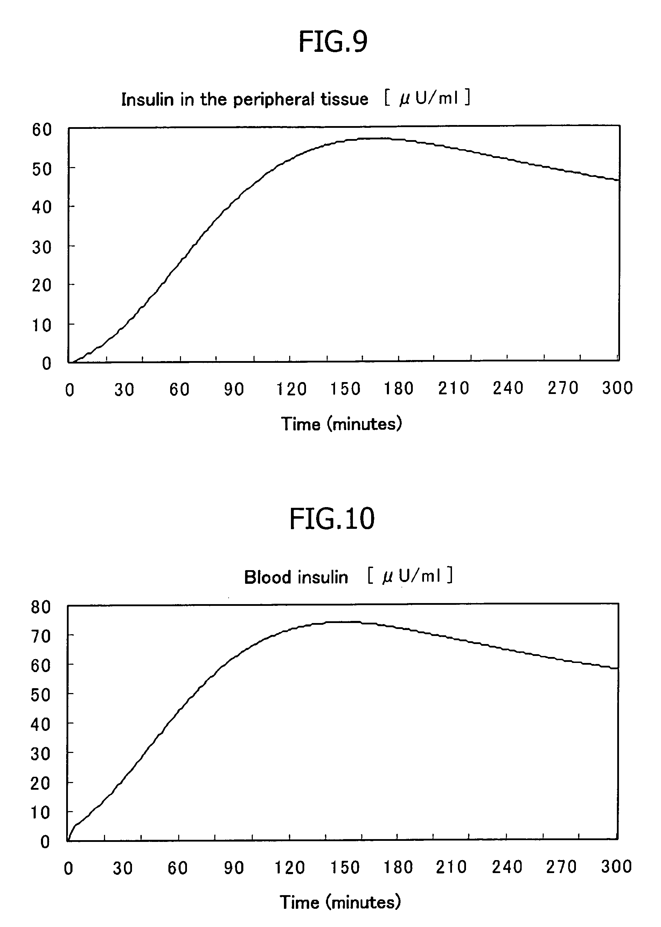

Subsequently, CPU110a makes display 120 display the time-series data of insulin concentration 10 and blood glucose level 6 which are provided by the simulation process S4 (step S5), and finishes the application program 140a. Then, in the step S5, the graph of which the vertical axis is assigned to the blood glucose level or the insulin concentration and the cross axis is assigned to time, as shown in figure 9 and figure 10, is displayed on the display 120.

In addition, for example, numerical value of the blood glucose level in each time and numerical value of the insulin concentration in each time may be displayed, and besides the time-series data of insulin concentration 10 and blood glucose level 6, other data treated by living body simulation model such as blood insulin concentration 35 may be displayed.

As mentioned above, the successively calculated blood glucose and insulin levels can be displayed on the display 120. In this way a user can easily confirm the results which simulate the organs of a living body. Furthermore, the present system also may be used as a subsystem for simulating body functions in medical systems, such as a diabetic diagnostic support system. In this case, the change over time in the calculated blood glucose and insulin levels may be transferred to other structural elements of the medical system to prepare, for example, diabetic diagnosis support information and the like, so as to provide highly reliable medical treatment information based on the blood glucose and insulin levels calculated by this system.

Calculations using the differential equations in the simulation process S4 can be accomplished using, for example, E-Cell (Public domain software created by Keio University), and MatLab (MathWorks, Inc.). Other calculation systems may also be used.

Shown below is an example of a change over time in blood glucose and insulin levels simulated using this system. This time the values in Table 1 were used as examples of the parameters of each block.

|  | Parameter | Value | Units |
|---|---|---|---|
| Pancreas | h | 48.5 | [mg/dl] |
|  | $\alpha$ | 70.3 | [min$^{-1}$] |
|  | $\beta$ | 5.14 | [($\mu$U/ml)·(mg/dl)$^{-1}$·min$^{-1}$] |
|  | M | 1.0 x10$^{-2}$ | [min$^{-1}$] |
| Liver | $G_b$ | 80 | [mg/dl] |
|  | $P_1$ | 1.16 x 10$^{-3}$ | [min$^{-1}$] |
|  | $P_2$ | 4.16 x 10$^{-6}$ | [($\mu$U/ml)$^{-1}$] |
|  | $G_{off}$ | 3.8 | [(mg/dl)·min$^{-1}$] |
|  | K | 9.64 x10$^{-2}$ |  |
| Insulin Kinetics | $A_1$ | 6.7 x10$^{-2}$ | [min$^{-1}$] |
|  | $A_2$ | 1.94 x 10$^{-1}$ | [min$^{-1}$] |
|  | $A_3$ | 4.38 x 10$^{-1}$ | [min$^{-1}$] |
|  | $A_4$ | 4.2 x 10$^{-2}$ | [min$^{-1}$] |
|  | $A_5$ | 2.2 x 10$^{-2}$ | [min$^{-1}$] |
|  | $A_6$ | 1.94 x10$^{-1}$ | [min$^{-1}$] |
| Peripheral Tissue | $K_1$ | 3.4 x 10$^{-2}$ | [min$^{-1}$] |
|  | $K_2$ | 4.7 x 10$^{-4}$ | [($\mu$U/ml)$^{-1}$·min$^{-1}$] |

Furthermore, the values of table 2 were used as examples of the initial values of the variables in calculations using the differential equations.

|  | Initial Value | Value | Units |
|---|---|---|---|
| Pancreas | X(0) | 2300 | [μU/ml] |
|  | Y(0) | 167.5 | [(μU/ml)·min$^{-1}$] |
|  | BG(0) | 80 | [mg/dl] |
| Insulin Kinetics | I$_1$(0) | 0 | [μU/ml] |
|  | I$_2$(0) | 0 | [μU/ml] |
|  | I$_3$(0) | 0 | [μU/ml] |

Furthermore, the glucose absorption rate from the alimentary canal used the values shown in Fig. 7

Among the simulation results under the above conditions, the change over time in blood glucose is shown n Fig. 8, the change over time in peripheral tissue insulin level 10 is shown in Fig. 9, and the change over time in blood insulin 35 and I$_3$(t) 34 are shown in Fig. 10. Furthermore, blood glucose clinical measurements are shown in Fig. 11. Results matching these physiological changes can be reproduced by executing a simulation using the present system.

As described above, the change over time in blood glucose and insulin levels accompanying glucose absorption can be reproduced in a form which closely matches physiological changes by using the present system. The models used in the present system are easily understandable from a medical perspective since they include blocks respectively corresponding to the pancreas, liver, insulin kinetics, and peripheral tissue as structural elements.

The foregoing detailed description and accompanying drawings have been provided by way of explanation and illustration, and are not intended to limit the scope of the appended claims. Many variations in the presently preferred embodiments illustrated herein will be obvious to one of ordinary skill in the art, and remain within the scope of the appended claims and their equivalents.

**Claims**

1.  A system for simulating a change over time in object material concentration in a living body, the system comprising:

    a biological simulation model representing the functions of organs in a living body related to the object material, and having a plurality of blocks corresponding to the organs respectively, which are mutually linked so as to enable the transference of data among blocks; and
    a calculation means for successively calculating the object material concentration in the living body to simulate the activity of the organs, and simulating the activity of the organs by driving the biological simulation model.

2.  The simulation system of claim 1, wherein the biological simulation model represents a function of an organ in a living body related to the absorption, accumulation, and metabolism of glucose and the secretion, transport, and action of insulin, and the blocks are related to the pancreas, liver, insulin kinetics, and peripheral tissue respectively, and the object material is at least one of insulin and glucose.

3.  The simulation system of Claim 2, wherein the pancreas block is a one-input one-output model in which the blood glucose level is the input and the insulin secretion is the output.

4.  The simulation system of Claim 2, wherein the liver block is a two-input two-output model in which the blood glucose level and the insulin secretion from the pancreas block are the inputs, and the insulin level and net glucose production from the liver are the outputs.

5.  The simulation system of Claim 2, wherein the insulin kinetic block is a one-input one-output model in which the insulin passed through the liver is the input, and the insulin level in peripheral tissue is the output.

6.  The simulation system of Claim 2, wherein the peripheral tissue block is three-input one-output model in which net glucose production from the liver (endogenous glucose), external glucose absorption (exogenous glucose), and insulin level are the inputs, and blood glucose level is the output.

7.  The simulation system of Claim 2, wherein the pancreas block is a model described a operation of the pancreas with a numerical expression including parameters and representing a function of the pancreas; and the parameter represent insulin production and secretion ability of the pancreas corresponding to the glucose level.

8. The simulation system of Claim 2, wherein the liver block is a model described a operation of the liver with a numerical expression including parameters and representing a function of the liver; and the parameter represent glucose uptake ability, glucose production ability, and amount of insulin used.

9. The simulation system of Claim 2, wherein the insulin kinetic block is a model described a operation of the insulin kinetic with a numerical expression including parameters and representing a function of the insulin kinetic; and the parameters represent the amount of insulin in the blood, and insulin acting ability near insulin target tissue.

10. The simulation system of Claim 2, wherein the peripheral tissue block is a model described a operation of the peripheral tissue with a numerical expression including parameters and representing a function of the peripheral tissue; and the parameters represent the insulin-independent glucose metabolism, and insulin-dependent glucose metabolism ability corresponding to the insulin level.

11. A computer-readable storage medium for recording a computer program for working a computer as a system for simulating a change over time in object material concentration in a living body, the computer program comprising:

   a step of driving, in a computer, a biological simulation model representing the functions of organs in a living body related to the object material to successively calculate the object material concentration in the living body; and
   wherein the biological simulation model have a plurality of blocks corresponding to the organs respectively , which are mutually linked so as to enable the transference of data among blocks.

12. The simulation system of Claim 1, wherein the biological simulation model represents a function of an organ in a living body related to the absorption, accumulation, and metabolism of glucose and the secretion, transport, and action of insulin, and the blocks are related to the pancreas, liver, insulin kinetics, and peripheral tissue respectively, and the object material is at least one of insulin and glucose.

13. The storage medium of Claim 12, wherein the pancreas block is a one-input one-output model in which the blood glucose level is the input and the insulin secretion is the output.

14. The storage medium of Claim 12, wherein the liver block is a two-input two-output model in which the blood glucose level and the insulin secretion from the pancreas block are the inputs, and the insulin level and net glucose production from the liver are the outputs.

15. The storage medium of Claim 12, wherein the insulin kinetic block is a one-input one-output model in which the insulin passed through the liver is the input, and the insulin level in peripheral tissue is the output.

16. The storage medium of Claim 12, wherein the peripheral tissue block is three-input one-output model in which net glucose production from the liver (endogenous glucose), external glucose absorption (exogenous glucose), and insulin level are the inputs, and blood glucose level is the output.

17. The storage medium of Claim 12, wherein the pancreas block is a model describing the parameters representing the function of the pancreas block and its operation using numerical expressions; and the parameters of the pancreas block represent insulin production and secretion ability of the pancreas corresponding to the glucose level.

18. The storage medium of Claim 12, wherein the liver block is a model describing the parameters representing the function of the liver block and its operation using numerical expressions; and the parameters of the liver block represent glucose uptake ability, glucose production ability, and amount of insulin used.

19. The storage medium of Claim 12, wherein the insulin kinetic block is a model describing the parameters representing the function of the insulin kinetic block and its operation using numerical expressions; and the parameters of the insulin kinetic block represent the amount of insulin in the blood, and insulin acting ability near insulin target tissue.

20. The storage medium of Claim 12, wherein the peripheral tissue block is a model describing the parameters representing the function of the peripheral block and its operation using numerical expressions; and the parameters of the peripheral block represent the insulin-independent glucose metabolism, and insulin-dependent glucose metabolism ability corresponding to the insulin level.

# FIG.1

EP 1 598 766 A2

# FIG.2

# FIG.3

# FIG.4

# FIG.5

# FIG.6

57

Blood glucose
BG

51

53

Net glucose
production from
the liver RGout

+ + +

1/S

K1

54

Glucose
absorption RG

52

K2

56

Peripheral tissue
insulinInsulin in the
55 peripheral tissue

# FIG.7

RG [(mg/dl)·min$^{-1}$]

Time (minutes)

# FIG.8

Blood glucose [mg/dl]

Time (minutes)

## FIG.9

Insulin in the peripheral tissue  [ $\mu$ U/ml ]

Time (minutes)

## FIG.10

Blood insulin  [ $\mu$ U/ml ]

Time (minutes)

# FIG.11

Blood glucose ［ｍｇ/ dl ］

Time (minutes)